# EUROPEAN PATENT APPLICATION

(11) **EP 4 674 372 A1**
(43) Date of publication of application: **07.01.2026**
(21) Application number: 24766469.1
(22) Date of filing: 06.03.2024
(51) Int. Cl.: A61B 34/20, A61B 90/20, A61B 34/00

(54) **IMAGE NAVIGATION METHOD AND SYSTEM FOR SURGICAL MICROSCOPE**

(30) Priority: 08.03.2023 CN 202310219513
(71) Applicant: Zumax Medical Co., Ltd., Jiangsu 215129 (CN)
(72) Inventor: WANG, Jilong, Suzhou, Jiangsu 215129 (CN); NADEAU, Bobby, Suzhou, Jiangsu 215129 (CN); HU, Zhuangzhuang, Suzhou, Jiangsu 215129 (CN); LI, Jianyue, Suzhou, Jiangsu 215129 (CN); QIU, Tao, Suzhou, Jiangsu 215129 (CN); HUANG, Bin, Suzhou, Jiangsu 215129 (CN); HE, Jin, Suzhou, Jiangsu 215129 (CN)
(74) Representative: Sun, Yiming
(86) International application number: PCT/CN2024/080308
(87) International publication number: WO 2024/183754

(57) **Abstract**

The present application discloses an image navigation method and system for a surgical microscope. The method comprises: fixing a surgical subject and a marker, and performing radiation imaging on the surgical subject and the marker to obtain a three-dimensional structural digital image; locating the marker via the image, establishing a coordinate system, and obtaining relative locations of a target affected part and the marker; setting an entry point and a path guide point of an instrument on the image to generate a navigation path, and transmitting data to a media signal processing apparatus; placing the target affected part and the marker in a field of view of a microscope, and transmitting an image in the field of view to the media signal processing apparatus; identifying, by the media signal processing apparatus, the marker to generate a matched three-dimensional space; and superposing a path image of corresponding coordinates on the image in the field of view according to the three-dimensional space to implement real-time navigation. The system comprises the microscope, the media signal processing apparatus, and the marker. The microscope comprises a microscope body and an image augmentation apparatus. The present application can precisely locate an entry point and an entry path of a surgical instrument, to guide a doctor or a related operator to complete a dental diagnosis and treatment surgery, thus improving the surgical precision.

## Description

### TECHNICAL FIELD

The present invention relates to the technical field of dental diagnosis and treatment, and in particular, to an image navigation method and system for a surgical microscope.

### BACKGROUND

Modern medicine has substantial progress in the fields such as *in vitro* diagnosis, microscopic treatment, medical imaging, and minimally invasive treatment, and cross-category and multi-discipline integrated-type diagnosis and treatment means emerge one after another. Owing to continuous development of medical imaging devices, medical imaging technologies are changing quickly, sub-disciplines such as computed tomography (CT), magnetic resonance (MR), interventional radiology, ultrasonics, and nuclear medicine in medical imaging are gradually established, and a medical imaging technology discipline is also gradually formed.

Medical imaging information is more sensitive, intuitive, specific, and of an early type. Image analysis develops from qualitative analysis to quantitative analysis, and develops from displaying diagnosis information to providing a solution of a surgical route. Image cameras and display develop from two-dimensional simulation to three-dimensional full digitalization. Image storage develops from hard copying using films to soft copying without films, and even image transmission networking. A single-imaging technology develops to a comprehensive-imaging technology.

To adapt to digitalization, networking, and integration of medical imaging, a viewpoint of integrating three professions, i.e., diagnosis, technologies, and engineering, needs to be established, and a single profession cannot complete functions of a modern medical imaging discipline.

For example, in a root canal therapy, a doctor needs to thoroughly open a medullary cavity to find and treat all root canals. In humans, there are usually 1-4 root canals in each tooth, and a posterior tooth has the most root canals. In a multi-root canal tooth, because of age-increasing changes, or deposition of restorative dentine, or denticles, or calcification of a pulp cavity, or changes in the root canal morphology, when it is not easy to detect a root canal orifice, it is necessary to understand and view an anatomic morphology of a pulp cavity from various directions and locations with the help of a three-dimensional anatomic morphology of a tooth. X-ray films photographed by a multi-angle projection method are used to learn and point out the number, shapes, locations, directions, and bending conditions of the root and the root canals, a relationship between the root and the crown, various possible variations of the root and the root canals in the anatomic morphology, and the like. Because the number of root canals of some tooth may be four, and complex cases such as lateral root canals, accessory root canals, apical ramifications, and apical furcations may also exist, even when observed under magnification, the root canals may be missed. A possible location of a root canal needs to be estimated. When necessary, a small amount of dentine may be removed from a site of a developmental groove where the root canal may be or is expected to be located by using a small round bur, and then any calcified area is attempted to be punched by using a sharp probe, so as to point out the root canal orifice to remove a dentine collar of the dental neck to expose the location of the root canal orifice. Namely, in case that calcification of a root canal orifice exists, a doctor needs to repeatedly probe possible locations, and unavoidably excessively remove healthy dentinal tissues.

Currently, preoperative periapical films are often used to help a doctor judge and determine the drill point and depth of a target tooth. First, the doctor needs to allow partial distractions to memorize the morphology of the root canal, and even suspend the surgery to re-observe the periapical films. Secondly, an error in human observation easily causes a deviation in an entry point of a bit, and the drill path and depth are determined according to experience of the doctor and cannot be accurately determined. In addition, an existing surgical navigation device is complex, and the preparation time before a surgery is long for a doctor, which severely affects the surgical efficiency.

Therefore, in view of the foregoing technical problems, new innovations are necessary.

### SUMMARY

An objective of the present invention is to at least solve one of the technical problems existing in the prior art, and therefore, provide an image navigation method and system for a surgical microscope, which are used to locate a target affected part and mark an entry point and an entry depth of a bit, so that a doctor can precisely perform a surgery.

The present invention provides an image navigation method for a surgical microscope, comprising the following steps:
S1: fixing a surgical subject and a marker, and performing radiation imaging on the surgical subject and the marker to obtain a three-dimensional structural digital image;
S2: locating the marker via the three-dimensional structural digital image, establishing a three-dimensional spatial coordinate system, and obtaining relative locations of a target affected part and the marker;
S3: setting an entry point and a path guide point of a surgical instrument on the three-dimensional structural digital image to generate a navigation path, and transmitting data of the navigation path to a media signal processing apparatus;
S4: placing the target affected part and the marker in a field of view of a surgical microscope, and transmitting an image in the field of view of the surgical microscope to the media signal processing apparatus;
S5: identifying, by the media signal processing apparatus, the marker to generate a three-dimensional space matching the image in the field of view of the surgical microscope; and
S6: superposing, by an image augmentation apparatus, a navigation path image of corresponding coordinates on the image in the field of view of the surgical microscope according to the three-dimensional space to implement real-time navigation.

Further, before S5, the media signal processing apparatus calibrates the marker to eliminate an error between an image received by the media signal processing apparatus and an actual image.

Further, the calibrating the marker comprises the following steps:
T1: photographing, by the media signal processing apparatus, a plurality of image pictures in the field of view of the surgical microscope via a camera module;
T2: detecting feature points in the pictures, and solving a homography matrix according to location information of the feature points and coordinates in the images;
T3: calculating internal parameters and external parameters by an analytic solution estimation method; and
T4: designing an optimization target according to a maximum likelihood estimation policy, and implementing parameter optimization to obtain high-precision parameters.

Further, the marker comprises a base and locators arranged on the base, the number of the locators is at least three, and calibration patterns are formed on the base.

In S1, the marker is arranged near the target affected part.

In S2, the three-dimensional structural digital image is opened by the media signal processing apparatus, and the three locators are sequentially located to establish the three-dimensional spatial coordinate system.

Further, the locators are metal balls.

Further, when each locator is located, central positions of the locator in three directions are selected simultaneously for locating.

Further, the calibration patterns are Aruco patterns.

In S5, the media signal processing apparatus extracts ID information of an aruco code in information of each of the Aruco patterns, establishes a world coordinate system based on the ID information of the Aruco code, and determines a pose of the marker in the field of view of the surgical microscope according to a corresponding relationship between image corners in corner coordinates and world corners in the world coordinate system, to generate a matched three-dimensional space.

In S6, the media signal processing apparatus registers the world coordinate system, a screen coordinate system, and the three-dimensional spatial coordinate system, and sends the navigation path image of corresponding coordinates in the three-dimensional space to the image augmentation apparatus to superpose the navigation path image on the image in the field of view of the surgical microscope.

Further, the media signal processing apparatus comprises a first media signal processing apparatus and a second media signal processing apparatus, the first media signal processing apparatus is communicatively connected to the second media signal processing apparatus, the second media signal processing apparatus is connected to the image augmentation apparatus via an optical adapter, and a camera module of the second media signal processing apparatus collects an image in the field of view of the surgical microscope via the optical adapter, and displays the collected image on the second media signal processing apparatus.

The first media signal processing apparatus receives data of the three-dimensional structural digital image of the surgical subject and the marker, and locates the marker via the three-dimensional structural digital image, to establish the three-dimensional spatial coordinate system.

The first media signal processing apparatus sets the entry point and the path guide point of the surgical instrument on the three-dimensional structural digital image to generate the navigation path, and transmits the data of the navigation path to the second media signal processing apparatus, the second media signal processing apparatus transmits the data of the navigation path to the image augmentation apparatus, the image augmentation apparatus converts the data of the navigation path into an optical image, and the optical image is superposed in the field of view of the surgical microscope to form a superposed image.

Further, S6 comprises: superposing, by the image augmentation apparatus, the correspondingly matched three-dimensional structural digital image on the image in the field of view of the surgical microscope according to the three-dimensional space.

Further, the image augmentation apparatus comprises a light splitting structure, the image in the field of view of the surgical microscope is displayed on the media signal processing apparatus in real time via the light splitting structure, and the image in the field of view of the surgical microscope comprises an image of the target affected part and an image of the marker.

The present invention further provides an image navigation system for a surgical microscope, comprising a surgical microscope, a media signal processing apparatus, and a marker, where the surgical microscope comprises a microscope body and an image augmentation apparatus, the image augmentation apparatus is arranged on the microscope body, the image augmentation apparatus comprises a light splitting structure, an image in a field of view of the surgical microscope is displayed on the media signal processing apparatus in real time via the light splitting structure, and the image augmentation apparatus is communicatively connected to the media signal processing apparatus.

The marker is arranged near a target affected part, and the media signal processing apparatus receives a three-dimensional structural digital image of a surgical subject and the marker, locates the marker via the three-dimensional structural digital image, establishes a three-dimensional spatial coordinate system, and obtains relative locations of the target affected part and the marker.

An entry point and a path guide point of a surgical instrument are set on the three-dimensional structural digital image to generate a navigation path, the media signal processing apparatus transmits data of the navigation path to the image augmentation apparatus, and the image augmentation apparatus converts the data of the navigation path into an optical image, and the optical image is superposed in a main optical path of the microscope body to form a superposed image in the field of view of the surgical microscope.

Further, the image augmentation apparatus comprises a projection module and a superposing lens, the projection module is communicatively connected to the media signal processing apparatus, the superposing lens is arranged on the main optical path of the microscope body, the projection module is configured to receive the data of the navigation path transmitted by the media signal processing apparatus, and convert the received data of the navigation path into an optical image, the superposing lens superposes the optical image transmitted by the projection module in the main optical path of the microscope body to form a superposed image, and the superposed image can be observed via a binocular tube of the surgical microscope.

Further, the media signal processing apparatus comprises a first media signal processing apparatus and a second media signal processing apparatus, the first media signal processing apparatus is communicatively connected to the second media signal processing apparatus, the second media signal processing apparatus is connected to the image augmentation apparatus via an optical adapter, and a camera module of the second media signal processing apparatus collects an image in the field of view of the surgical microscope via the optical adapter, and displays the collected image on the second media signal processing apparatus.

The first media signal processing apparatus receives the three-dimensional structural digital image of the surgical subject and the marker, and locates the marker via the three-dimensional structural digital image, to establish the three-dimensional spatial coordinate system.

The first media signal processing apparatus sets the entry point and the path guide point of the surgical instrument on the three-dimensional structural digital image to generate the navigation path, and transmits the data of the navigation path to the second media signal processing apparatus, and the second media signal processing apparatus transmits the data of the navigation path to the image augmentation apparatus.

Compared with the prior art, the image navigation method and system for a surgical microscope of the present application at least have one or more of the following beneficial effects:
The image navigation method and system for a surgical microscope of the present application can precisely locate the entry point and the entry path of the surgical instrument, to guide a doctor or a related operator to complete a dental diagnosis and treatment surgery, thus improving the surgical precision. Also, the overall solution is simple and easy in operation, so that the preparation time before a surgery can be effectively reduced, and the surgical efficiency can be improved. Generally, an existing navigation system for a surgical microscope needs a dedicated navigator, which is relatively large in volume, and easily blocks the field of view of the surgical microscope. In contrast, the present application does not need a dedicated navigator, has low cost, high efficiency, convenience in use, and fewer instruments, and shortens the setting and diagnosis time for a surgery.

The marker is designed to improve the navigation accuracy and reduce complexity of the entire system.

A dual-optical-path superposition design may be used, to ensure that when an operator observes through the binocular tube, both eyes can observe a superposed image, so that it is more comfortable and convenient for the operator to observe the image. A digital micromirror device (DMD) projection module is preferably used to replace a conventional organic light-emitting diode (OLED) screen for projection. Due to a difference in illumination principles, the brightness of the DMD projection module can reach hundreds of times the brightness of the OLED screen, thereby effectively solving the problem of a poor contrast ratio of the OLED screen when the screen is highlighted.

### BRIEF DESCRIPTION OF THE DRAWINGS

FIG. 1 is a flowchart of an image navigation method according to an embodiment of the present application;
FIG. 2 is a schematic structural diagram of a marker according to an embodiment of the present application;
FIG. 3 is a mounting location diagram of the marker according to an embodiment of the present application;
FIG. 4 to FIG. 6 are screenshots of a software interface when three locators are located according to an embodiment of the present application;
FIG. 7 is a screenshot of a software interface when an entry point is set according to an embodiment of the present application;
FIG. 8 is a screenshot of a software interface when a path guide point is set according to an embodiment of the present application;
FIG. 9 is a schematic diagram of a navigation path displayed through simulation in a second media signal processing apparatus according to an embodiment of the present application;
FIG. 10 is a schematic diagram of set locations of two media signal processing apparatuses according to an embodiment of the present application;
FIG. 11 is a schematic diagram of a set location of a second media signal processing apparatus on a surgical microscope according to an embodiment of the present application;
FIG. 12 is a screenshot of a software interface when the marker is calibrated according to an embodiment of the present application;
FIG. 13 is a schematic diagram of image processing light spots of the three locators and the entry point in a surgery according to an embodiment of the present application;
FIG. 14 is a schematic diagram of a cone beam CT (CBCT) superposed image when the marker is laterally adhered according to an embodiment of the present application;
FIG. 15 shows a set location of an image augmentation apparatus on a microscope body according to an embodiment of the present application;
FIG. 16 is a schematic diagram of a cross-sectional structure of the image augmentation apparatus according to an embodiment of the present application;
FIG. 17 is a schematic diagram of the direction of an internal light beam of the image augmentation apparatus according to an embodiment of the present application;
FIG. 18 is a schematic diagram of the directions of a light beam when a first superposing lens superposes and splits the light beam according to an embodiment of the present application;
FIG. 19 is a three-dimensional schematic structural diagram of the image augmentation apparatus according to an embodiment of the present application;
FIG. 20 is a top view schematic structural diagram of the image augmentation apparatus according to an embodiment of the present application;
FIG. 21 is a rear view schematic structural diagram of the image augmentation apparatus according to an embodiment of the present application; and
FIG. 22 is a schematic diagram of the operation status of an operator in a surgery according to an embodiment of the present application.

1-Surgical microscope, 11-Microscope body, 12-Image augmentation apparatus, 121-Projection module, 1211-DMD lens surface, 1212-Filter lens, 1213-Prism group, 1214-Projection correction lens, 122-First lens combination, 1221-First lens group, 12211-First lens, 12212-Second lens, 1222-Second lens group, 123-First reflecting lens, 124-Second lens combination, 1241-Third lens group, 12411-Fourth lens, 12412-Fifth lens, 125-Beam-splitting lens, 126-Second reflecting lens, 127-First superposing lens, 128-Second superposing lens, 129-Shell, 1291-Shell body, 1292-Backplate, 1293-Power jack, 1294-Power switch, 1295-Connection interface, 12951-TYPE-C interface, 12952-HDMI interface, 1296-Beam-splitting interface, 1297-Lens tube interface, 130-Diaphragm, 131-Diaphragm adjustment apparatus, 132-Lens holder, 13-Binocular tube, 2-Marker, 21-Base, 211-Seat, 212-Pattern plate, 22-Locator, 23-Calibration pattern, 3-First media signal processing apparatus, 4-Second media signal processing apparatus, 5-Optical adapter, 6-Holder, 7-Navigation path image, 8-Target affected part, and 9-Surgical instrument.

### DETAILED DESCRIPTION

To further explain the technical means used in the present invention for achieving the intended objectives and the effects thereof, specific implementation manners, structures, features, and effects of the present invention are described in detail below with reference to the accompanying drawings and preferred embodiments.

### Embodiment

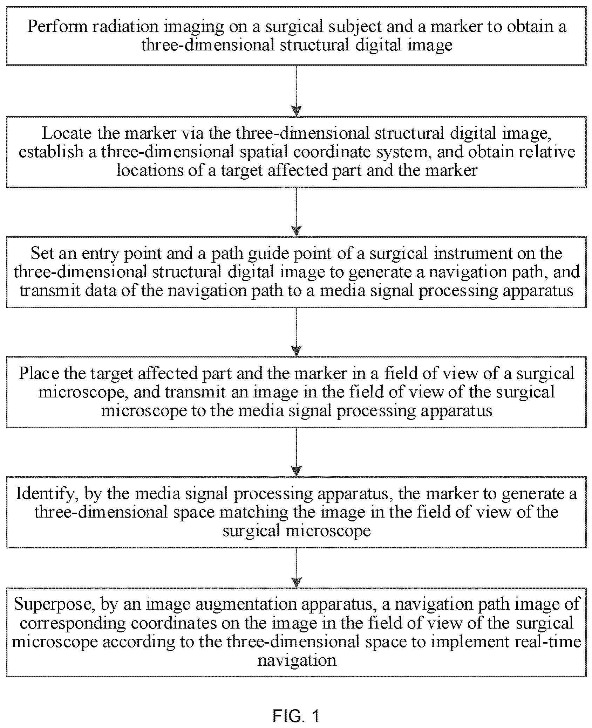

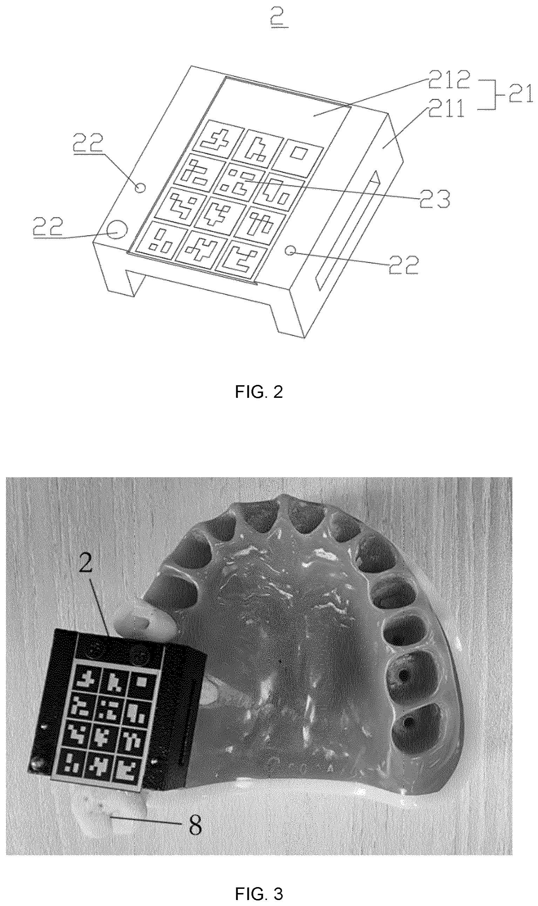

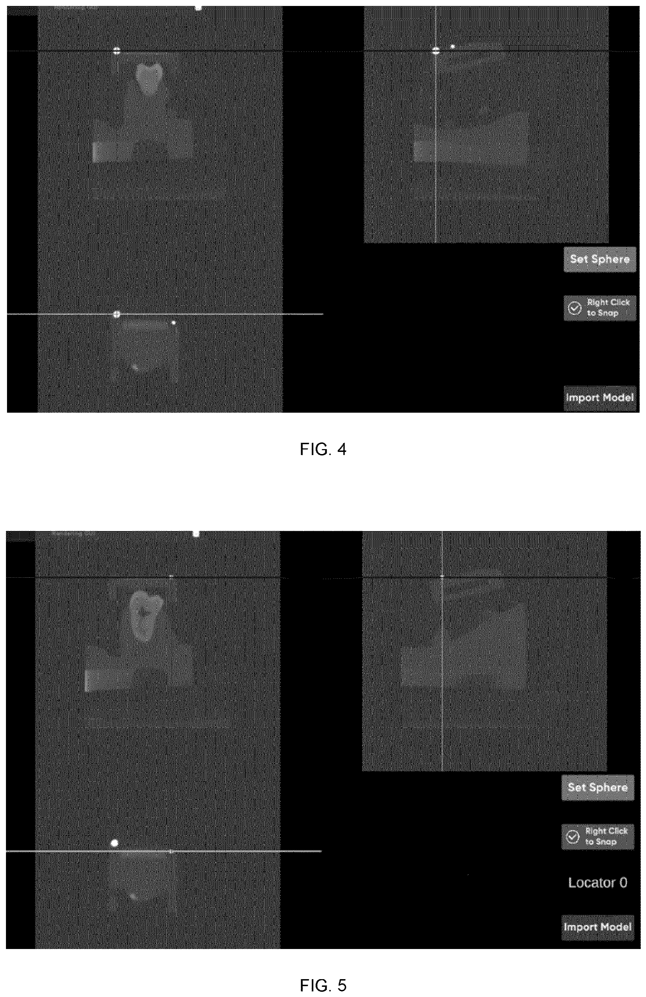

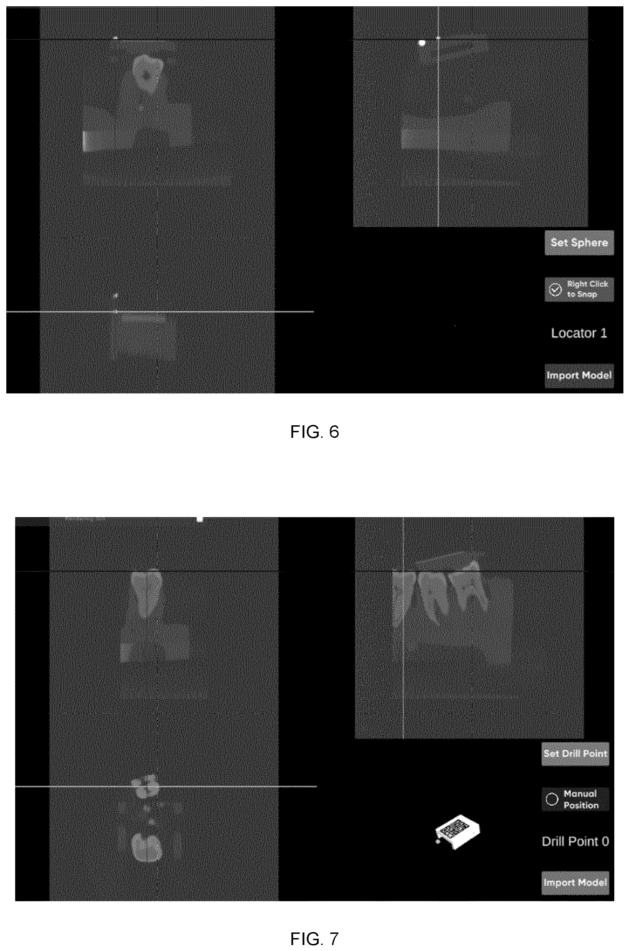

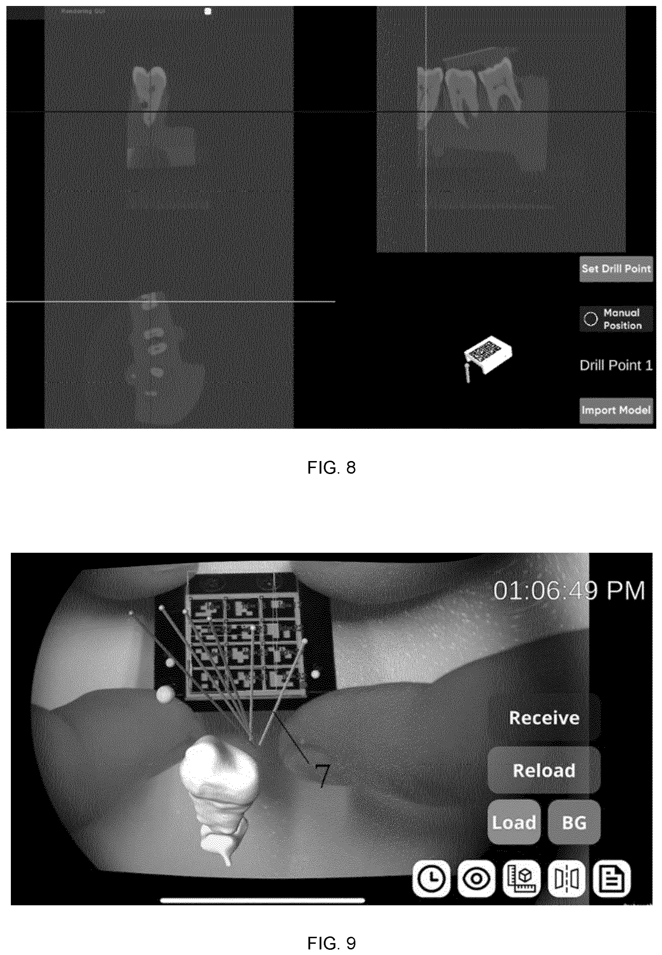

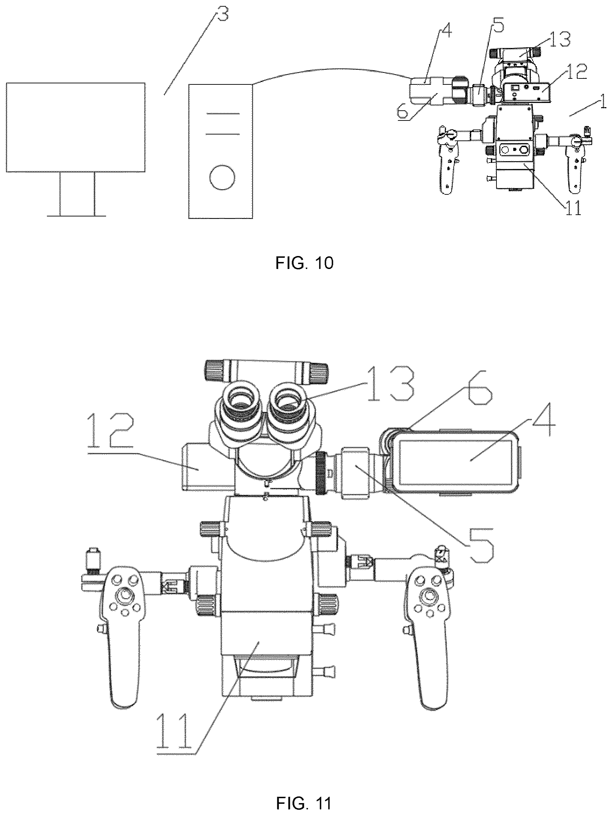

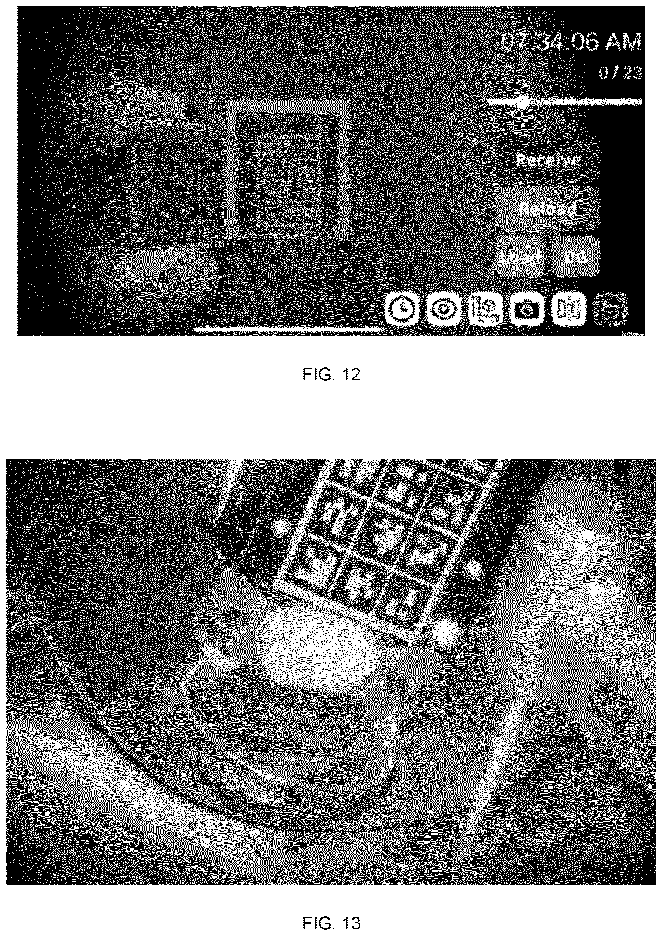

This embodiment provides an image navigation method for a surgical microscope. As shown in FIG. 1, the image navigation method mainly comprises the following steps:
S1: A surgical subject and a marker 2 are fixed, and radiation imaging is performed on the surgical subject and the marker 2 to obtain a three-dimensional structural digital image. Before a surgery, the marker 2 is first arranged near a target affected part 8. The marker 2 comprises a base 21 and locators 22 arranged on the base 21. The number of the locators 22 is at least three. Calibration patterns 23 are formed on the base 21. The locators 22 preferably comprise a first locator, a second locator, and a third locator. At least two locators 22 have different sizes, and the three locators 22 are not in a straight line. A first plane formed by one end of each of the three locators 22 intersects with a second plane formed by the other end of each of the three locators 22. The first locator and the second locator that have relatively large volumes are located on one side of the calibration patterns 23, and the third locator is located on the other side of the calibration patterns 23. A distance between the second locator and the third locator is 2 to 8 times a distance between the second locator and the first locator. A distance between the first locator and the second locator is in a range of 2 mm to 8 mm. A distance between the first locator and the third locator is in a range of 8 mm to 33 mm. A distance between the second locator and the third locator is in a range of 8 mm to 35 mm. The diameter of the first locator is in a range of 1 mm to 8 mm. The diameter of the second locator is in a range of 1 mm to 6 mm. The diameter of the third locator is in a range of 1 mm to 6 mm. In this way, the accuracy of navigation can be improved, and complexity of an entire system can be reduced. The locators 22 are preferably metal balls, so that when radiation imaging is performed, the three-dimensional structural digital image comprises image information of the three locators 22. Using a dental surgery as an example, before the surgery, the marker 2 is first fixed at a target affected part 8, namely, near a target tooth. For example, the marker 2 may use a structural design shown in FIG. 2, and comprise a base 21 and three metal ball locators 22. One of the metal balls is relatively large, and the other two metal balls are relatively small and have the same size. The base 21 comprises a base 211 and a pattern plate 212. The calibration patterns 23 are formed on the pattern plate 212. One side of the base 211 is provided with a fixing groove, and the other side is provided with an accommodating groove. The pattern plate 212 is fixedly arranged in the accommodating groove. The pattern plate 212 and the base 211 may be fixedly connected by fasteners such as bolts, or may be fixedly connected by gluing or the like. Certainly, the pattern plate 212 may also be integrally formed with the base 211, namely, one side of the base 21 is provided with the fixing groove, and the other side of the base 21 is provided with the calibration patterns 23. During use, the base 21 is mounted on a patient's teeth via the fixing groove, and is adhered and fixed by a special adhesive. The three locators 22 are fixedly arranged around the calibration patterns 23, for example, as shown in FIG. 2, arranged on two sides of the pattern plate 212. The locators 22 may be fixed in a manner that, for example, as shown in FIG. 2, circular grooves are formed at set locations on the base 211, and then the locators 22 are fixed in the corresponding circular grooves by gluing or the like. Then, CBCT is performed on the affected part and the marker plate, to obtain a three-dimensional structural digital image exhibiting the dental hard tissue, the root canal morphology, the metal balls in the marker plate, and the like. It is to be noted that the foregoing is merely a preferred solution. During specific implementation, the shapes, sizes, set locations, and fixing manners of the locators 22 are not limited, and may be flexibly designed as required. In addition, the fixed location of the marker 2 is not limited, but it needs to be ensured that when radiation imaging such as CBCT is performed, no artifact is generated, and the locators 22 can have sufficient features. In addition, it needs to be ensured that a target tooth and other important information are not blocked in the field of view of the microscope, and detailed information of the calibration patterns 23 on the marker 2 can be clearly displayed, namely, the calibration patterns 23 of a suitable size can be displayed, as shown in FIG. 3. The calibration patterns 23 are preferably Aruco patterns. As shown in FIG. 2, binary coding inside a pattern of this type makes an algorithm very robust, and allows a possibility of applying an error detection and correction technology, so that a particular marker 2 can be converted into three-dimensional coordinates. Certainly, the calibration patterns 23 are not limited to Aruco patterns, and may also be other patterns comprising plane patterns and three-dimensional patterns. The number of patterns needs to be selected such that the ratio of the number of patterns in the transverse direction to that in the longitudinal direction is close to 1:1. On the premise that clear identification of details can be ensured, precision can be improved as the number of patterns increases during both calibration and navigation.
S2: The marker 2 is located via the three-dimensional structural digital image. A three-dimensional spatial coordinate system is established. Relative locations of a target affected part 8 and the marker 2 are obtained. In this step, the first media signal processing apparatus 3, e.g., a computer, may receive data of the three-dimensional structural digital image of the surgical subject and the marker 2, open the three-dimensional structural digital image, and then sequentially locate the three locators 22 to establish the three-dimensional spatial coordinate system. Central positions of one locator 22 in three directions need to be selected simultaneously for locating to reduce errors. The computer has built-in software. Via the software, the three-dimensional structural digital image may be presented on a computer screen in a manner of three views. An operator operates the computer to select a certain point on the three views simultaneously to determine three-dimensional coordinates of the point. For example, as shown in FIG. 4 to FIG. 6, relative spatial coordinates of the three locators 22 may be determined by selecting the central positions of each locator 22 in three directions simultaneously by using the software, to establish the three-dimensional spatial coordinate system, and obtain location coordinates of all locations of the target affected part 8 and all locations of the marker 2 in the three-dimensional spatial coordinate system.
S3: An entry point and a path guide point of a surgical instrument 9 are set on the three-dimensional structural digital image to generate a navigation path. Data of the navigation path is transmitted to a second media signal processing apparatus 4. The second media signal processing apparatus is, for example, a mobile terminal such as a mobile phone or a tablet computer, and is in communicatively connected with the computer in S2. The surgical instrument 9 is, for example, a bit used in a root canal therapy. In this step, the entry point and the path guide point are set in a manner as in S2. The operator selects the entry point and the path guide point in three directions via the software according to an operation requirement, as shown in FIG. 7 and FIG. 8, to determine three-dimensional coordinates of the two points separately. Then the software generates the navigation path according to the three-dimensional coordinates of the two points, namely, a straight line starting from the entry point and passing through the path guide point, as shown in FIG. 9. A plurality of navigation paths are schematically shown in FIG. 9. In addition, it is to be noted that the first media signal processing apparatus 3 and the second media signal processing apparatus 4 may be the same device, for example, the same mobile terminal. The locators 22 may be located and the entry point and the path guide point may be set in S2 and S3 by directly embedding corresponding software in the mobile terminal.
S4: The target affected part 8 and the marker 2 are placed in a field of view of a surgical microscope 1. An image in the field of view of the surgical microscope 1 is transmitted to the second media signal processing apparatus 4, namely, a mobile terminal. The mobile terminal is connected to an image augmentation apparatus 12 via an optical adapter 5. As shown in FIG. 10 and FIG. 11, the mobile terminal is shown as a mobile phone. For the specific principle of the optical adapter 5, reference may be made to patent application No. 201720275596.5. For the specific principle of the image augmentation apparatus 12, reference may be made to patent application No. 202121362228.7. The optical adapter 5 is connected to a beam-splitting interface 1296 of the image augmentation apparatus 12. The mobile terminal is fixedly connected to the optical adapter 5 via a holder 6. An image in the field of view of the surgical microscope 1 is subjected to light splitting by a beam-splitting structure, namely, a beam-splitting lens, in the image augmentation apparatus 12, is incident on the optical adapter 5, and then is collected by a camera module of the mobile terminal. Then, the mobile terminal may display the collected image on a screen of the mobile terminal, so as to display the image in the field of view of the surgical microscope 1 in real time. The image in the field of view of the surgical microscope 1 comprises the image of the target affected part 8 and the image of the marker 2.
S5: The second media signal processing apparatus 4 identifies the marker 2 to generate a three-dimensional space matching the image in the field of view of the surgical microscope 1. Before this step, the second media signal processing apparatus 4 needs to calibrate the marker 2, to eliminate an error between an image received by the second media signal processing apparatus 4 and an actual image, namely, eliminate an error from a real object to the surgical microscope 1 and from the surgical microscope 1 to the mobile phone, thereby improving precision of the spatial location of the three-dimensional object. The calibrating the marker 2 comprises the following steps: T1: The second media signal processing apparatus 4 photographs a plurality of image pictures in the field of view of the surgical microscope 1 via a camera module, as shown in FIG. 12. T2: Feature points in the pictures are detected. A homography matrix is solved according to location information of the feature points and coordinates in the images. T3: Internal parameters and external parameters are calculated by an analytic solution estimation method. T4: An optimization target is designed according to a maximum likelihood estimation policy, and parameter optimization is implemented to obtain high-precision parameters. After the marker 2 is calibrated, the second media signal processing apparatus 4 tracks the calibration patterns 23 on the marker 2 in real time, to determine the pose of the marker 2. Taking an Aruco pattern as an example, the second media signal processing apparatus 4 extracts ID information of an Aruco code in information of the Aruco pattern, establishes a world coordinate system based on the ID information of the Aruco code, and determines the pose of the marker 2 in the field of view of the surgical microscope 1 according to a corresponding relationship between image corners in corner coordinates and world corners in the world coordinate system, to generate a matched three-dimensional space.
S6: The image augmentation apparatus 12 superposes a navigation path image 7 of corresponding coordinates on the image in the field of view of the surgical microscope 1 according to the three-dimensional space to implement real-time navigation. After the three-dimensional space matching the image in the field of view of the surgical microscope 1 is generated, the relative location of the target affected part 8 in the three-dimensional space may be determined according to the location coordinates, obtained in S2, of all locations of the target affected part 8 and all locations of the marker 2 in the three-dimensional spatial coordinate system. The navigation path image 7 of the corresponding coordinates is loaded. Then, the second media signal processing apparatus 4 fuses the navigation path image 7 with the actual image in the field of view of the surgical microscope 1, for example, uses an AR kit development library to implement registration of the world coordinate system, a screen coordinate system, and the three-dimensional spatial coordinate system, and then transmits data of the navigation path image 7 of the corresponding coordinates in the three-dimensional space to a projection module 121 such as a display apparatus of the image augmentation apparatus 12. The projection module 121 converts the data of the navigation path into an optical image. The optical image is superposed in the field of view of the surgical microscope 1 via a superposing lens to form a superposed image, thereby implementing real-time navigation. The operator may control a motion path of the surgical instrument 9 according to the navigation path, so as to precisely treat the target affected part 8. In the field of view of the surgical microscope 1, the three locators 22 and the entry point are all preferably highlighted via light spots, as shown in FIG. 13, which may be more convenient for the operator to observe.

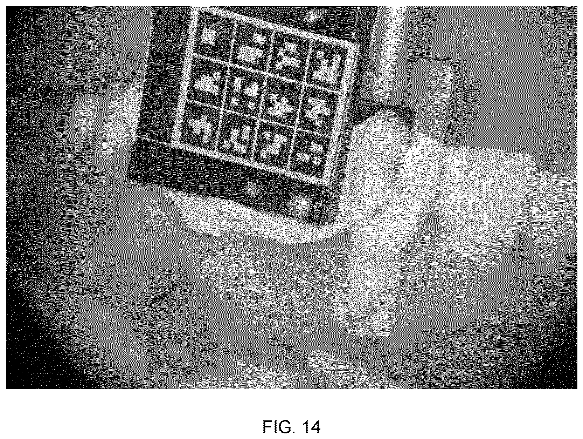

In addition, the image superposed in the field of view of the surgical microscope 1 is not limited to the navigation path image 7, and may also be a three-dimensional structural digital image obtained by radiation imaging. For example, as shown in FIG. 14, the marker 2 is laterally adhered to the teeth. The second media signal processing apparatus 4 identifies the pose of the marker 2, transmits data of a correspondingly matched CBCT three-dimensional structural digital image to the projection module 121, and finally superposes the digital image in the field of view of the surgical microscope 1. The operator can precisely locate the dental pulps of the teeth with the support of the CBCT superposed image, to complete cutting procedures.

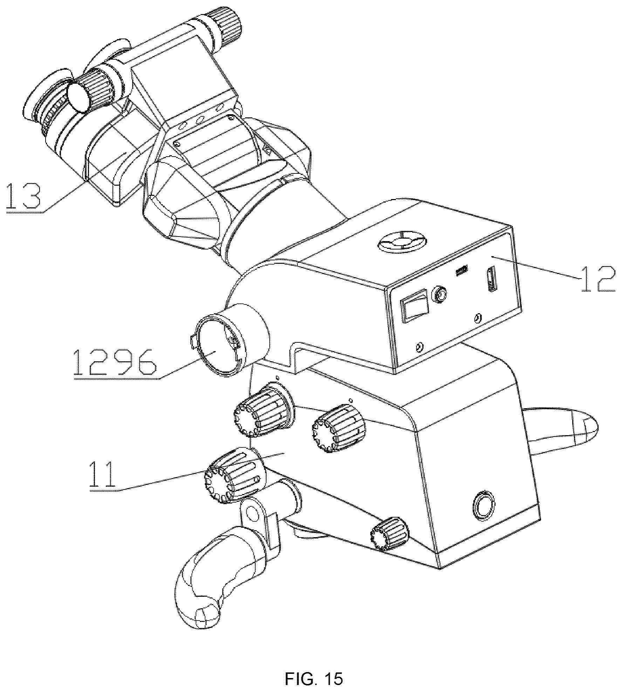

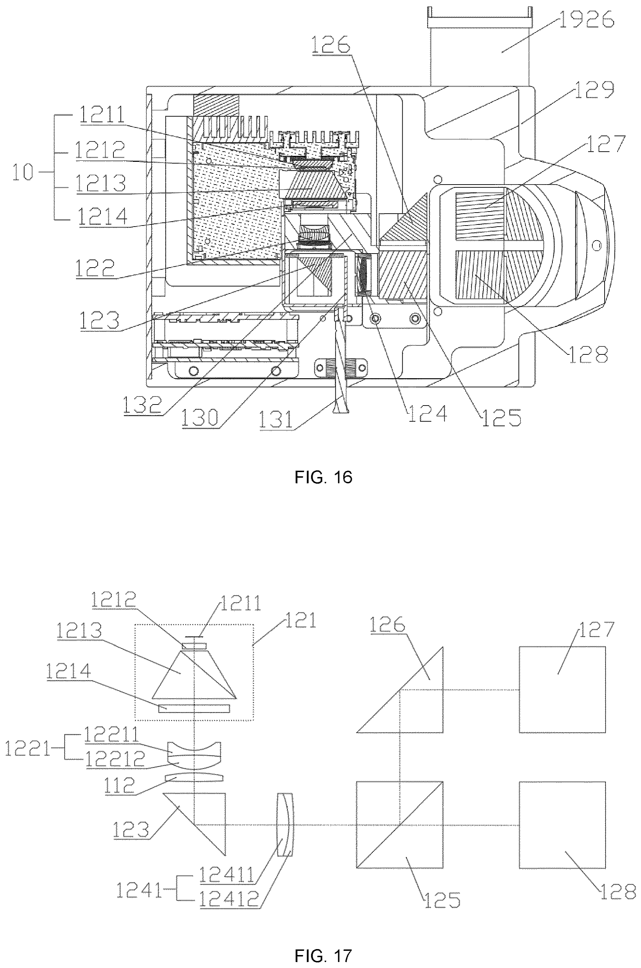

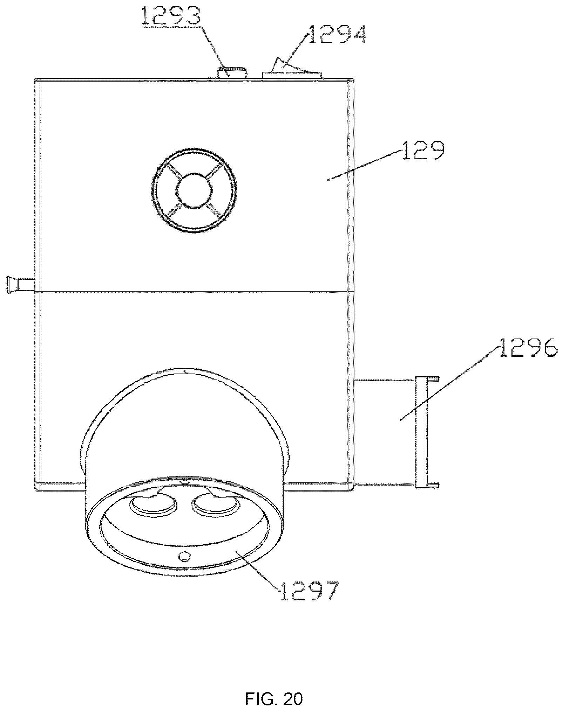

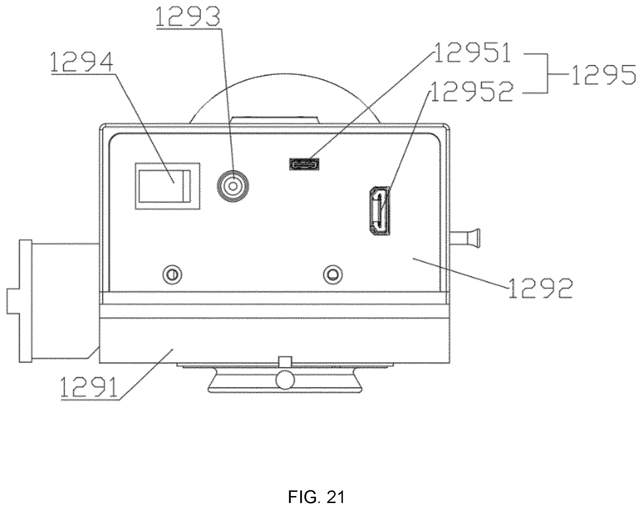

This embodiment further provides an image navigation system for the aforementioned image navigation method, comprising a surgical microscope 1, a media signal processing apparatus, and a marker 2. The surgical microscope 1 comprises a microscope body 11 and an image augmentation apparatus 12. The image augmentation apparatus 12 is arranged on the microscope body 11, as shown in FIG. 10, FIG. 11, and FIG. 15. The image augmentation apparatus 12 comprises a shell 129, as well as the projection module 121 and a beam-splitting structure arranged inside the shell 129. The beam-splitting structure is the beam-splitting lens. When the image augmentation apparatus 12 is arranged on the microscope body 11, the beam-splitting lens is located in a main optical path of the microscope body 11. A beam-splitting interface 1296 is arranged on the shell 129. After an image in the field of view of the surgical microscope 1 is subjected to light splitting by the beam-splitting lens of the image augmentation apparatus 12, one part is incident on a binocular tube 13, and the other part is emitted out through the beam-splitting interface 1296. The beam-splitting lens is used to split light, and is further used as a superposing lens used to superpose the incident images. A light beam emitted by the projection module 121 is reshaped in an optical path, and then the light beam is incident on the superposing lens and is superposed in the main optical path of the microscope body 11, so as to form a superposed image. For the specific principle of the image augmentation apparatus 12, reference may be made to patent application No. 202121362228.7. It is to be noted that the patent application discloses merely superposing an image in a single optical path, and the image augmentation apparatus 12 in this embodiment may also use two beam-splitting lenses as superposing lenses. For example, as shown in FIG. 16, one more beam-splitting lens and one more reflecting lens are arranged in an optical path, so as to split a light beam emitted by the projection module 121 into two parts, and superpose the two parts on two optical paths of the microscope body 11. Therefore, when the binocular tube 13 is used for observation, both eyes can observe the superposed image. In addition, the beam-splitting interface 1296 of the image augmentation apparatus 12 is preferably arranged corresponding to the beam-splitting lens used to superpose an image, so that an image emitted through the beam-splitting interface 1296 is a superposed image. Next, the dual-path superposition image augmentation apparatus 12 is described with reference to FIG. 15 to FIG. 22 as follows:
The image augmentation apparatus 12 comprises the projection module 121, a first lens combination 122, a first reflecting lens 123, a second lens combination 124, a beam-splitting lens 125, a second reflecting lens 126, a first superposing lens 127, and a second superposing lens 128, as shown in FIG. 16. The projection module 121, the first lens combination 122, the first reflecting lens 123, the second lens combination 124, the beam-splitting lens 125, the second reflecting lens 126, the first superposing lens 127, and the second superposing lens 128 are all arranged in the shell 129. Further, the first lens combination 122, the first reflecting lens 123, the second lens combination 124, the beam-splitting lens 125, and the second reflecting lens 126 are mounted in the shell 129 via lens holders 132 separately. The first reflecting lens 123 and the second reflecting lens 126 are preferably right-angle prisms. The beam-splitting lens 125, the first superposing lens 127, and the second superposing lens 128 are preferably beam-splitting prisms.

The projection module 121, the first lens combination 122, and the first reflecting lens 123 are sequentially arranged in the same optical path. The first reflecting lens 123 and the second lens combination 124 are arranged in an incident optical path of the beam-splitting lens 125. The second reflecting lens 126 is arranged in a first emergent optical path of the beam-splitting lens 125. The first superposing lens 127 and the second reflecting lens 126 are arranged in the same optical path. The second superposing lens 128 is arranged in a second emergent optical path of the beam-splitting lens 125. When the image augmentation apparatus 12 is arranged on the microscope body 21, as shown in FIG. 15, the first superposing lens 127 is located in a first main optical path of the microscope body 21, and the second superposing lens 128 is located in a second main optical path of the microscope body 21. As shown in FIG. 17, after the light beam emitted by the projection module 121 passes through the first lens combination 122, the light beam is reflected and rotated by 90 degrees by the first reflecting lens 123, then passes through the second lens combination 124, and is incident on the beam-splitting lens 125. The beam-splitting lens 125 splits a part of the light beam to the second reflecting lens 126. The second reflecting lens 126 reflects and rotates the part of the light beam by 90 degrees to the first superposing lens 127, and the part of the light beam is superposed in the first main optical path of the microscope body 21 to form a composite optical image. The beam-splitting lens 125 splits the other part of the light beam to the second superposing lens 128, and the other part of the light beam is superposed in the second main optical path of the microscope body 21 to form a composite optical image. Then, the operator can observe, by the binocular tube 22 of the surgical microscope 2, the superposed image with the content, projected by the projection module 121, superposed.

A diaphragm 19 may be further arranged between the first reflecting lens 123 and the second lens combination 124, so as to block or transmit an optical path, thereby enabling or disabling a superposed image as required. Specifically, as shown in FIG. 16, a diaphragm adjustment apparatus 131 is arranged in the shell 129. The diaphragm 19 can be driven to move between a blocking position and an open position by adjusting the diaphragm adjustment apparatus 131. When the diaphragm 19 is located in the blocking position, the diaphragm 19 is located in the same optical path as the first reflecting lens 123 and the second lens combination 124, and the diaphragm 19 blocks a light beam that is incident on the second lens combination 124 after passing through the first reflecting lens 123. The adjustment manner of the diaphragm 19 may be horizontal insertion, or rotational insertion. Any adjustment manner capable of inserting the diaphragm 19 into and retracting the diaphragm out of an optical path falls within the protection scope of the present application.

The projection module 121 is preferably a DMD projection module, and comprises a DMD lens surface 1211, a filter lens 1212, a prism group 1213, and a projection correction lens 1214, as shown in FIG. 16 and FIG. 17. The brightness of the DMD projection module is provided by a plurality of LED light sources of different colors, is reflected by the DMD lens surface 1211 to the projection lens group, sequentially passes through the filter lens 1212, the prism group 1213, and the projection correction lens 1214, is incident on the first lens combination 122, and is reshaped by the light path to form a projection pattern. Due to a difference in illumination principles, the brightness of a DMD projection solution can reach hundreds of times that of an OLED screen.

The first lens combination 122 comprises a first lens group 1221 and a second lens group 1222, and the second lens combination 124 comprises a third lens group 1241, as shown in FIG. 17. The first lens group 1221 is a cemented doublet group having negative optical power, and comprises a first lens 12211 and a second lens 12212. The second lens group 1222 is a third lens having positive optical power. The first lens 12211 is located on a side of the second lens 12212 that faces toward the projection correction lens 1214, and the third lens is located on a side of the second lens 12212 that faces away from the first lens 12211. The third lens group 1241 is a cemented doublet group having positive optical power, and comprises a fourth lens 12411 and a fifth lens 12412. The fourth lens 12411 is located on a side of the fifth lens 12412 that faces toward the first reflecting lens 123. Further, the first lens combination 122 and the second lens combination 124 conform to the following formula:
25<(*f*_{*G*2}+*f*_{*G*3})/(*f*_{*G*3}*-f*_{*G*2})<35, *f*_{*G*2} is the focal length of the second lens group 1222, and *f*_{*G*3} is the focal length of the third lens group 1241;
*φ*_{*G*3}/(*T*_{*L*4}*+T*_{*L*5})*>*3.5, *φ*_{*G*3} is the effective aperture of the third lens group 1241, *T*_{*L*4} is the thickness of the fourth lens 12411, and *T*_{*L*5} is the thickness of the fifth lens 12412; and
2.5*<T*_{*L*2}/*T*_{*L*1} <3.5, *T*_{*L*1}, is the thickness of the first lens 12211, and *T*_{*L*2} is the thickness of the second lens 12212.

As shown in the following table:

| Surface number | Radius | Thickness | Nd | Vd | Half aperture |
|---|---|---|---|---|---|
| 1 | ∞ | 1.35 | | | 4.8 |
| 2 | ∞ | 1.1 | 1.52 | 64.2 | 2.5 |
| 3 | ∞ | 0.4 | | | 2.5 |
| 4 | ∞ | 10 | 1.52 | 64.2 | 3 |
| 5 | ∞ | 1.3 | | | 9 |
| 6 | ∞ | 1.6 | 1.52 | 58.6 | 8 |
| 7 | ∞ | 7.9 | | | 8 |
| 8 | -6.979 | 1 | 1.58 | 41.5 | 5.5 |
| 9 | 50.785 | 3 | 1.61 | 60.6 | 5.5 |
| 10 | -10.107 | 0.3 | | | 5.5 |
| 11 | 29.929 | 1.8 | 1.62 | 57 | 5.5 |
| 12 | -137.902 | 2.9 | | | 6 |
| 13 | ∞ | 13 | 1.52 | 64.2 | 6 |
| 14 | ∞ | 11.26 | | | 6.5 |
| 15 | 108.929 | 2.5 | 1.72 | 43.7 | 6.5 |
| 16 | -17.846 | 1 | 1.58 | 59.5 | 6.5 |
| 17 | -60.563 | | | | 6.5 |

In the table, the radius is the curvature radius of the lens surface; the thickness is the distance between the lens surface and another lens surface at the central positions along the optical path; the Nd is the refractive index of d light (having a wavelength of 589.3 nm) in optical glass; the Vd is the Abbe number; and the half aperture is a half of the effective aperture of the lens surface. Some parameters in the table are explained and described with reference to FIG. 17 as follows:
The surface 1 is the DMD lens surface 1211, and the thickness is a distance between the surface and an upper lens surface of the filter lens 1212 at the central positions.
The surface 2 is the upper lens surface of the filter lens 1212, and the thickness is a distance between the surface and a lower lens surface of the filter lens 1212 at the central positions.
The surface 3 is the lower lens surface of the filter lens 1212, and the thickness is a distance between the surface and an upper lens surface of a prism combination 103 at the central positions.
The surface 4 is the upper lens surface of the prism combination 103, and the thickness is a distance between the surface and a lower lens surface of the prism combination 103 at the central positions.
The surface 5 is the lower lens surface of the prism combination 103, and the thickness is a distance between the surface and an upper lens surface of the projection correction lens 1214 at the central positions.
The surface 6 is the upper lens surface of the projection correction lens 1214, and the thickness is a distance between the surface and a lower lens surface of the projection correction lens 1214 at the central position.
The surface 7 is the lower lens surface of the projection correction lens 1214, and the thickness is a distance between the surface and an upper lens surface of the first lens group 1221 at the central positions.
The surface 8 is the upper lens surface of the first lens group 1221, and the thickness is a distance between the surface and a cemented surface between the first lens 12211 and the second lens 12212 at the central positions.
The surface 9 is the cemented surface between the first lens 12211 and the second lens 12212, and the thickness is a distance between the surface and a lower lens surface of the first lens group 1221 at the central positions.
The surface 10 is the lower lens surface of the first lens group 1221, and the thickness is a distance between the surface and an upper lens surface of the second lens group 1222 at the central positions.
The surface 11 is the upper lens surface of the second lens group 1222, and the thickness is a distance between the surface and a lower lens surface of the second lens group 1222 at the central positions.
The surface 12 is the lower lens surface of the second lens group 1222, and the thickness is a distance between the surface and an upper lens surface of the first reflecting lens 123 at the central positions.
The surface 13 is the upper lens surface of the first reflecting lens 123, and the thickness is a distance between the surface and a reflecting surface of the first reflecting lens 123 and a distance between the reflecting surface of the first reflecting lens 123 and a right lens surface of the first reflecting lens 123, respectively at the central positions.
The surface 14 is the right lens surface of the first reflecting lens 123, and the thickness is a distance between the surface and a left lens surface of the third lens group 1241 at the central positions.
The surface 15 is the left lens surface of the third lens group 1241, and the thickness is a distance between the surface and a cemented surface between the fourth lens 12411 and the fifth lens 12412 at the central positions.
The surface 16 is the cemented surface between the fourth lens 12411 and the fifth lens 12412, and the thickness is a distance between the surface and a right lens surface of the third lens group 1241 at the central positions.
The surface 17 is a right lens surface of the third lens group 1241.

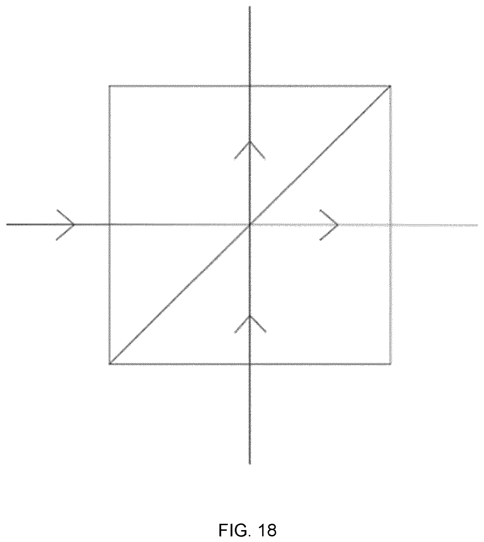

In a further embodiment, one side of the shell 129 is provided with a light-splitting interface 1296 matching the first superposing lens 127 or the second superposing lens 128. For example, the light-splitting interface is arranged on a light-splitting side of the first superposing lens 127, and a part of a light beam of a superposed image formed by superposing in the first main optical path of the microscope body 21 in the surgical microscope 2 may be emitted out through the light-splitting interface 1296. As shown in FIG. 18, a left light beam is a light beam emitted in by the projection module 121; a lower light beam is an incident light beam along the first main optical path of the microscope body 21; an upper light beam is a part of a light beam of a superposed image, split and emitted by the first superposing lens 127, to the binocular tube 22; and a right light beam is the other part of the light beam of the superposed image, split and emitted by the first superposing lens 127, to the light-splitting interface 1296. A mobile terminal having a camera module, for example, a mobile phone or a tablet computer, is connected to the light-splitting interface 1296, so that an image in the field of view of the surgical microscope 2 can be collected and recorded.

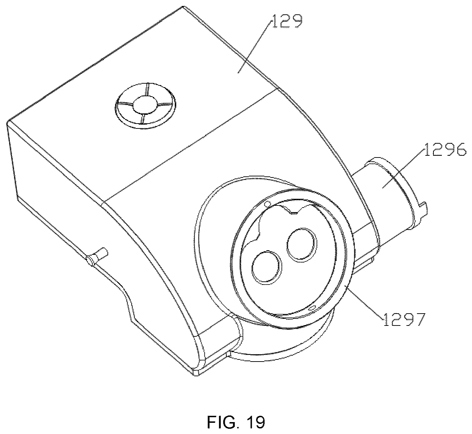

The shell 129 preferably comprises a shell body 1291 and a back plate 1292. Components such as a power jack 1293, a power switch 1294, and a connection interface 1295 are arranged on the back plate 1292, as shown in FIG. 19 to FIG. 21. The power jack 1293, the power switch 1294, and the connection interface 1295 are electrically connected to the projection module 121 respectively. The power jack 1293 is used to supply power to the projection module 121, and the power switch 1294 is used to control on-off of the projection module 121. The connection interface 1295 may be, for example, a TYPE-C interface 12951, and may be used to charge a mobile terminal such as a mobile phone or a tablet computer or other digital devices. The connection interface 1295 may be, for example, an HDMI interface 12952, and may be used to input data information to the projection module 121.

The media signal processing apparatus preferably comprises a first media signal processing apparatus 3 and a second media signal processing apparatus 4. The first media signal processing apparatus 3 is communicatively connected to the second media signal processing apparatus 4. The first media signal processing apparatus is preferably a computer. The second media signal processing apparatus 4 is preferably a mobile terminal such as a mobile phone or a tablet computer. The projection module 121 is communicatively connected to the second media signal processing apparatus 4. The marker 2 is arranged near a target affected part 8, so that an image in the field of view of the surgical microscope 1 comprises an image of the target affected part 8 and an image of the marker 2. The second media signal processing apparatus 4 is preferably connected to the image augmentation apparatus 12 via an optical adapter 5. For the specific principle of the optical adapter 5, reference may be made to patent application No. 201720275596.5. The optical adapter 5 is connected to the light-splitting interface 1296 of the image augmentation apparatus 12. An image in the field of view of the surgical microscope 1 is subjected to light splitting by a beam-splitting structure, namely, a beam-splitting prism, in the image augmentation apparatus 12, is incident on the optical adapter 5 through the light-splitting interface 1296, and then is collected by a camera module of the second media signal processing apparatus 4. Then, the second media signal processing apparatus 4 may display the collected image on a screen of the second media signal processing apparatus, so as to display the image in the field of view of the surgical microscope 1 in real time.

The first media signal processing apparatus 3 receives a three-dimensional structural digital image of a surgical subject and the marker 2, locates the marker 2 via the three-dimensional structural digital image, establishes a three-dimensional spatial coordinate system, and obtains relative locations of the target affected part 8 and the marker 2. The operator sets the entry point and the path guide point of the surgical instrument 9 by the first media signal processing apparatus on the three-dimensional structural digital image to generate the navigation path, and transmits the data of the navigation path to the second media signal processing apparatus 4. The second media signal processing apparatus 4 identifies the pose of the marker 2, and transmits data of a matched navigation path to the projection module 121 of the image augmentation apparatus 12. The specific principle is described in detail in the aforementioned method, and the descriptions thereof are omitted here. The projection module 121 converts the received data of the navigation path into an optical image, and then the optical image emitted by the projection module 121 is superposed in the main optical path of the microscope body 11 via the superposing lens to form a superposed image, thereby implementing real-time navigation.

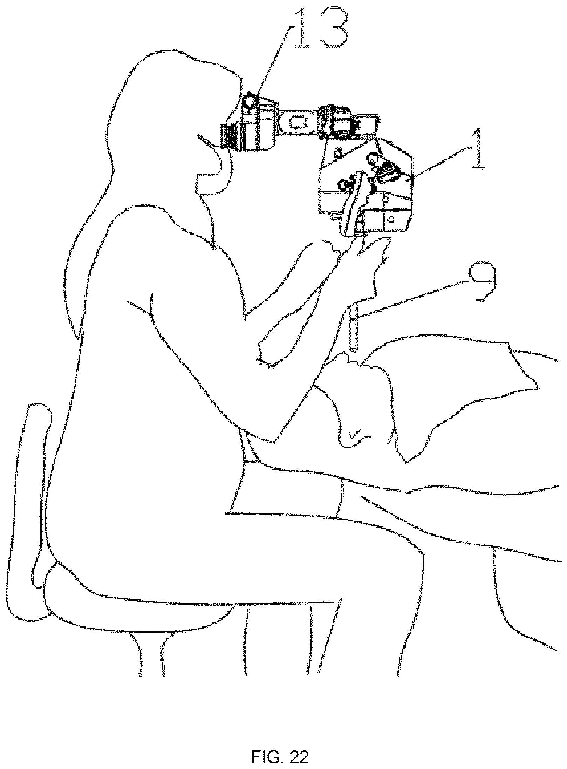

The binocular tube 13 is arranged on the microscope body 11, and preferably, is arranged on the image augmentation apparatus 12. Namely, the binocular tube 13 is preferably arranged on the microscope body 11 via the image augmentation apparatus 12. For example, as shown in FIG. 19, a lens tube interface 1297 used to fix the binocular tube 13 is arranged at an exit opening of the image augmentation apparatus 12. After the media signal processing apparatus transmits information data to the projection module 121, the projection module 121 converts the received information data into an optical image, and the optical image is superposed in a first main optical path of the microscope body 11 and a second main optical path of the microscope body 11 via the first superposing lens 127 and the second superposing lens 128 respectively, to form a superposed image. Then, the operator can observe, by the binocular tube 13, the superposed image with the content, projected by the projection module 121, superposed, as shown in FIG. 22.

The terms "include", "comprise", or any other variants thereof are intended to cover a non-exclusive inclusion herein, so that not only are those listed elements comprised, but also other elements which are not expressly listed are comprised.

The directional terms such as front, rear, above, and below involved are defined according to the locations of parts in the accompanying drawings and locations between the parts herein, and are merely for the purpose of expressing the technical solutions clearly and conveniently. It is to be understood that the use of the directional terms should not limit the protection scope of the present application.

The foregoing embodiments and features in the embodiments may be combined with each other without conflict herein.

The foregoing descriptions are merely preferred embodiments of the present invention, but are not intended to limit the present invention. Any modification, equivalent replacement, improvement, or the like made within the spirit and principle of the present invention shall fall within the protection scope of the present invention.

## Claims

1. An image navigation method for a surgical microscope, comprising the following steps:
S1: fixing a surgical subject and a marker (2), and performing radiation imaging on the surgical subject and the marker (2) to obtain a three-dimensional structural digital image;
S2: locating the marker (2) via the three-dimensional structural digital image, establishing a three-dimensional spatial coordinate system, and obtaining relative locations of a target affected part (8) and the marker (2);
S3: setting an entry point and a path guide point of a surgical instrument (9) on the three-dimensional structural digital image to generate a navigation path, and transmitting data of the navigation path to a media signal processing apparatus;
S4: placing the target affected part (8) and the marker (2) in a field of view of a surgical microscope (1), and transmitting an image in the field of view of the surgical microscope (1) to the media signal processing apparatus;
S5: identifying, by the media signal processing apparatus, the marker (2) to generate a three-dimensional space matching the image in the field of view of the surgical microscope (1); and
S6: superposing, by an image augmentation apparatus (12), a navigation path image (7) of corresponding coordinates on the image in the field of view of the surgical microscope (1) according to the three-dimensional space to implement real-time navigation.

2. The image navigation method for a surgical microscope according to claim 1, wherein before S5, the media signal processing apparatus calibrates the marker (2) to eliminate an error between an image received by the media signal processing apparatus and an actual image.

3. The image navigation method for a surgical microscope according to claim 2, wherein the calibrating the marker (2) comprises the following steps:
T1: photographing, by the media signal processing apparatus, a plurality of image pictures in the field of view of the surgical microscope (1) via a camera module;
T2: detecting feature points in the pictures, and solving a homography matrix according to location information of the feature points and coordinates in the images;
T3: calculating internal parameters and external parameters by an analytic solution estimation method; and
T4: designing an optimization target according to a maximum likelihood estimation policy, and implementing parameter optimization to obtain high-precision parameters.

4. The image navigation method for a surgical microscope according to claim 1, wherein the marker (2) comprises a base (21) and locators (22) arranged on the base (21), the number of the locators (22) is at least three, and calibration patterns (23) are formed on the base (21);
in S1, the marker (2) is arranged near the target affected part (8); and
in S2, the three-dimensional structural digital image is opened by the media signal processing apparatus, and the three locators (22) are sequentially located to establish the three-dimensional spatial coordinate system.

5. The image navigation method for a surgical microscope according to claim 4, wherein the locators (22) are metal balls.

6. The image navigation method for a surgical microscope according to claim 4, wherein when each locator (22) is located, central positions of the locator (22) in three directions are selected simultaneously for locating.

7. The image navigation method for a surgical microscope according to claim 4, wherein the calibration patterns (23) are Aruco patterns;
in S5, the media signal processing apparatus extracts ID information of an Aruco code in information of each of the Aruco patterns, establishes a world coordinate system based on the ID information of the Aruco code, and determines a pose of the marker (2) in the field of view of the surgical microscope (1) according to a corresponding relationship between image corners in corner coordinates and world corners in the world coordinate system, to generate a matched three-dimensional space; and
in S6, the media signal processing apparatus registers the world coordinate system, a screen coordinate system, and the three-dimensional spatial coordinate system, and sends the navigation path image (7) of corresponding coordinates in the three-dimensional space to the image augmentation apparatus (12) to superpose the navigation path image on the image in the field of view of the surgical microscope (1).

8. The image navigation method for a surgical microscope according to claim 2, wherein the media signal processing apparatus comprises a first media signal processing apparatus (3) and a second media signal processing apparatus (4), the first media signal processing apparatus (3) is communicatively connected to the second media signal processing apparatus (4), the second media signal processing apparatus (4) is connected to the image augmentation apparatus (12) via an optical adapter (5), and a camera module of the second media signal processing apparatus (4) collects an image in the field of view of the surgical microscope (1) via the optical adapter (5), and displays the collected image on the second media signal processing apparatus (4);
the first media signal processing apparatus (3) receives data of the three-dimensional structural digital image of the surgical subject and the marker (2), and locates the marker (2) via the three-dimensional structural digital image, to establish the three-dimensional spatial coordinate system; and
the first media signal processing apparatus (3) sets the entry point and the path guide point of the surgical instrument (9) on the three-dimensional structural digital image to generate the navigation path, and transmits the data of the navigation path to the second media signal processing apparatus (4), the second media signal processing apparatus (4) transmits the data of the navigation path to the image augmentation apparatus (12), the image augmentation apparatus (12) converts the data of the navigation path into an optical image, and the optical image is superposed in the field of view of the surgical microscope (1) to form a superposed image.

9. The image navigation method for a surgical microscope according to claim 1, wherein S6 further comprises:
superposing, by the image augmentation apparatus (12), the correspondingly matched three-dimensional structural digital image on the image in the field of view of the surgical microscope (1) according to the three-dimensional space.

10. The image navigation method for a surgical microscope according to claim 1, wherein the image augmentation apparatus (12) comprises a light splitting structure, the image in the field of view of the surgical microscope (1) is displayed on the media signal processing apparatus in real time via the light splitting structure, and the image in the field of view of the surgical microscope (1) comprises an image of the target affected part (8) and an image of the marker (2).

11. An image navigation system for a surgical microscope, comprising a surgical microscope (1), a media signal processing apparatus, and a marker (2), wherein the surgical microscope (1) comprises a microscope body (11) and an image augmentation apparatus (12), the image augmentation apparatus (12) is arranged on the microscope body (11), the image augmentation apparatus (12) comprises a light splitting structure, an image in a field of view of the surgical microscope (1) is displayed on the media signal processing apparatus in real time via the light splitting structure, and the image augmentation apparatus (12) is communicatively connected to the media signal processing apparatus;
the marker (2) is arranged near a target affected part (8), and the media signal processing apparatus receives a three-dimensional structural digital image of a surgical subject and the marker (2), locates the marker (2) via the three-dimensional structural digital image, establishes a three-dimensional spatial coordinate system, and obtains relative locations of the target affected part (8) and the marker (2);
an entry point and a path guide point of a surgical instrument (9) are set on the three-dimensional structural digital image to generate a navigation path, the media signal processing apparatus transmits data of the navigation path to the image augmentation apparatus (12), the image augmentation apparatus (12) converts the data of the navigation path into an optical image, and the optical image is superposed in a main optical path of the microscope body (11) to form a superposed image in the field of view of the surgical microscope (1).

12. The image navigation system for a surgical microscope according to claim 11, wherein the image augmentation apparatus (12) comprises a projection module (121) and a superposing lens, the projection module (121) is communicatively connected to the media signal processing apparatus, the superposing lens is arranged on the main optical path of the microscope body (11), the projection module (121) is configured to receive the data of the navigation path transmitted by the media signal processing apparatus, and convert the received data of the navigation path into an optical image, the superposing lens superposes the optical image transmitted by the projection module (121) in the main optical path of the microscope body (11) to form a superposed image, and the superposed image can be observed via a binocular tube (13) of the surgical microscope (1).

13. The image navigation system for a surgical microscope according to claim 12, wherein the media signal processing apparatus comprises a first media signal processing apparatus (3) and a second media signal processing apparatus (4), the first media signal processing apparatus (3) is communicatively connected to the second media signal processing apparatus (4), the second media signal processing apparatus (4) is connected to the image augmentation apparatus (12) via an optical adapter (5), and a camera module of the second media signal processing apparatus (4) collects an image in the field of view of the surgical microscope (1) via the optical adapter (5), and displays the collected image on the second media signal processing apparatus (4);
the first media signal processing apparatus (3) receives the three-dimensional structural digital image of the surgical subject and the marker (2), and locates the marker (2) via the three-dimensional structural digital image, to establish the three-dimensional spatial coordinate system; and
the first media signal processing apparatus (3) sets the entry point and the path guide point of the surgical instrument (9) on the three-dimensional structural digital image to generate the navigation path, and transmits the data of the navigation path to the second media signal processing apparatus (4), and the second media signal processing apparatus (4) transmits the data of the navigation path to the image augmentation apparatus (12).
